# EUROPEAN PATENT SPECIFICATION

(11) **EP 1 683 873 B1**
(45) Date of publication and mention of the grant of the patent: **14.01.2009**
(21) Application number: 06001259.8
(22) Date of filing: 20.01.2006
(51) Int. Cl.: C12Q 1/68

(54) **Method of removing nucleic acid amplification inhibitor from biological sample and a Micro-PCR system**
Verfahren zur Entfernung von Nukleinsäureamplifikationsinhibitoren in einer biologischen Probe und Mikro-PCR System
Méthode permettant d'enlever un inhibiteur d'amplification d'acides nucléiques d'un échantillon et système de Micro-PCR

(30) Priority: 20.01.2005 KR 2005005538
(43) Date of publication of application: 26.07.2006
(73) Proprietor: Samsung Electronics Co., Ltd., Suwon-si, Gyeonggi-Do (KR)
(72) Inventor: Kim, Kui-hyun, Daejeon-si (KR); Kim, Young-a, 245-1804 Hwanggol Maeul, Suwon-si, Gyeonggi-do (KR); Min, Jun-hong, Jukjeon-dong, Yongin-si, Gyeonggi-do (KR); Lee, Jeong-gun, Seoul (KR); Lee, In-ho, 505-1201 Geumhwa Maeul, Giheung-gu, Yongin-si, Gyeonggi-do (KR)
(74) Representative: Grünecker, Kinkeldey, Stockmair & Schwanhäusser Anwaltssozietät

(56) References cited:
- WO-A-89/08705
- WO-A-03/033740
- US-A- 5 646 263

## Description

### 1. Field of the Invention

The present invention relates to a method of removing an amplification inhibitor from a nucleic acid sample, and more particularly, to a method of efficiently removing an amplification inhibitor prior to amplification for detection of nucleic acids in a sample, in particular, in serum.

### 2. Description of the Related Art

In molecular biological and medical experiments, detection of specific DNAs in a sample, in particular, in a serum sample is often carried out. In this case, the most problematic factor for detection of serum DNAs is the presence of substances inhibiting the detection of the serum DNAs. That is, during amplification reaction (e.g., PCR amplification) for DNA detection, several substances including serum proteins may adsorb DNAs or interact with DNAs, thereby resulting in inhibition of PCR amplification. In particular, it is known that serum proteins have a considerable amplification inhibitory effect.

These other substances except serum DNAs may also serve as PCR inhibitory substances.

In addition, with respect to a serum sample analysis in a nanoscale biosensor, big serum proteins may cause a severe noise and easily block nano-sized pores.

In this regard, efficient removal of proteins and other mixtures in a sample, in particular, in serum is required.

A nucleic acid extraction method using QlAamp UltraSens Virus Kit (Qiagen, inc.) is currently used for removal of proteins and other mixtures in serum. According to the nucleic acid extraction method, cells are lysed and precipitated in a buffer AC of the kit. Then, the precipitate is resuspended in a buffer AR containing protease K to digest proteins. Then, a buffer AB is added and the cell lysate is washed twice to elute pure RNAs or DNAs. The nucleic acid extraction method is very complicated by total 16 steps, a process duration of one hour or more, and the use of six types of reagents.

Therefore, it is required the development of a method of simply removing an amplification inhibitor in a nucleic acid sample, in particular, in serum, in the absence of a harmful reagent within a short time.

### SUMMARY OF THE INVENTION

The present invention provides a method of simply removing an amplification inhibitor from a nucleic acid sample.

The present invention also provides a LIP (Lab In Package) capable of performing an amplification reaction simultaneously with or subsequently to removing an amplification inhibitor from a nucleic acid sample.

According to an aspect of the present invention, there is provided a method of removing a nucleic acid amplification inhibitor from a biological sample, the method including contacting the biological sample to a carboxyl group-coated solid support.

The method may further include filtering the solid support contacted to the biological sample.

The nucleic acid amplification may be PCR.

According to another aspect of the present invention, there is provided a micro-PCR system including: a sample pretreatment chamber including a carboxyl group-coated solid support; a PCR chamber; and a channel connecting the sample pretreatment chamber and the PCR chamber.

The channel may include a valve.

The solid support may be in the form of a plate, a bead, or a pillar.

The solid support may be made of glass, silicone, or polymer.

The polymer may be selected from the group consisting of polyethylene, polypropylene, polyacrylate, polyurethane, and polystyrene.

### BRIEF DESCRIPTION OF THE DRAWINGS

The above and other features and advantages of the present invention will become more apparent by describing in detail exemplary embodiments thereof with reference to the attached drawings in which:
FIG. 1 is a comparative graph illustrating the concentration of IgG before and after addition of M270 beads to a serum sample;
FIG. 2 is a graph illustrating PCR results for serum samples treated with M270 beads, M280 beads, and polystyrene beads;
FIG. 3 is a Scanning Electron Microscopic (SEM) image showing a morphological variation of carboxyl group-coated beads M270 added to a serum sample;
FIG. 4 is a graph illustrating PCR results for serum samples treated with M270 and a Qiagen kit;
FIG. 5 is a diagram illustrating a carboxyl group-coated bead according to an embodiment of the present invention;
FIG. 6 is a schematic view illustrating a Nanopore detection system according to an embodiment of the present invention;
FIG. 7 is a schematic view illustrating a PCR system according to embodiment of the present invention; and
FIG. 8 is a schematic enlarged sectional view of a sample pretreatment chamber according to an embodiment of the present invention.

### DETAILED DESCRIPTION OF THE INVENTION

A method of removing an amplification inhibitor from a nucleic acid sample and a PCR system according to the present invention will now be described more fully with reference to the accompanying drawings, in which exemplary embodiments of the invention are shown.

The present invention provides a method of removing a nucleic acid amplification inhibitor from a sample prior to nucleic acid amplification, the method including contacting the sample to a carboxyl group-coated solid support.

The present inventors found that a carboxyl group had adsorptivity to a nucleic acid amplification inhibitor in a sample, and completed the present invention.

There are no limitations on the sample provided that is a biological sample. For example, the sample may be blood, serum, urine, sperm, saliva, tissue culture, or cell culture.

All kinds of means capable of contacting a carboxyl group to a target sample for nucleic acid amplification can be within the scope of the present invention. According to an aspect of the present invention, the sample is contacted to the carboxyl group-coated solid support.

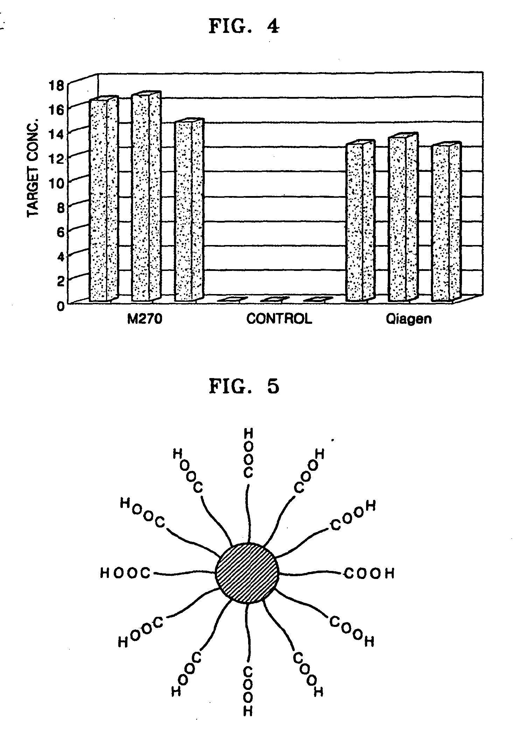

The carboxyl group-coated solid support is not particularly limited. For example, a carboxyl group-coated plate, bead, pillar, etc. may be used. A schematic diagram of a carboxyl group-coated bead is illustrated in FIG. 5. The above-mentioned plate, bead, pillar, etc. are not particularly limited provided that are made of a material capable of being coated with a carboxyl group. For example, the material capable of being coated with a carboxyl group may be glass, silicone, polymer, etc.

A carboxyl group coating method varies according to the type of the solid support to be coated. For this, a carboxyl group coating method commonly known in the art may be used. Alternatively, a commercially available carboxyl group-coated material may also be used.

After the sample is contacted to the carboxyl group-coated solid support, the solid support on which an amplification inhibitor is adsorbed is removed, and only a supernatant is used for nucleic acid amplification. The solid support on which an amplification inhibitor is adsorbed may be removed by centrifugation or filtration with a filter.

The resultant sample after centrifugation or filtration is used for nucleic acid amplification. Various nucleic acid amplification methods known in the art may be used. Nucleic acid amplification may be performed by PCR, LCR (Ligase Chain Reaction), or RCA (Rolling Circle Amplification), but is not limited thereto. A method of the present invention can be efficiently adopted as a pretreatment process for PCR.

The "PCR" refers to polymerase chain reaction and is well known in the art. Generally, PCR is performed in an amplification reaction solution containing a primer pair, a template, polymerase, and dNTPs by repeated cycles of the following three steps: denaturation, annealing, and extension. In the denaturation step, double-stranded nucleic acids are separated into two single strands at a denaturation temperature. In the annealing step, two primers of the primer pair are each bound to the complementary opposite strands at an annealing temperature. In the extension step, primer extension occurs by the polymerase at an extension temperature. The amplification reaction solution may vary according to the type of amplification reaction. Generally, however, the amplification reaction solution is not particularly limited provided that can allow polymerase to induce nucleic acid polymerization. A method of removing a nucleic acid amplification inhibitor according to the present invention is simple, cost effective, and time non-consuming, relative to a conventional technique. For example, the conventional QlAamp UltraSens Virus Kit extraction method (Qiagen) is very complicated by a process duration of one hour or more, total 16 purification steps, and the use of six types of reagents. On the other hand, according to a method of the present invention, most of amplification inhibitors are removed within 5 minutes. Furthermore, since only centrifugation or filtration is performed for a nucleic acid sample contacted with a carboxyl group, a process is very simplified. In addition, since a toxic reagent is not used, amplification reaction is not adversely affected.

As described above, according to a method of the present invention, an amplification inhibitor can be simply removed without using an additional process or apparatus. The method of the present invention can be applied to all kinds of amplification reactions anywhere at any time by those of ordinary skill in the art.

Furthermore, as will be described in the following Examples, a method of the present invention exhibits more excellent amplification inhibitor removal effect relative to a conventional technique.

To execute the above method, the present invention also provides a micro-PCR system including therein a sample pretreatment chamber containing a carboxyl group-coated solid support, a PCR chamber, and a channel connecting the sample pretreatment chamber and the PCR chamber.

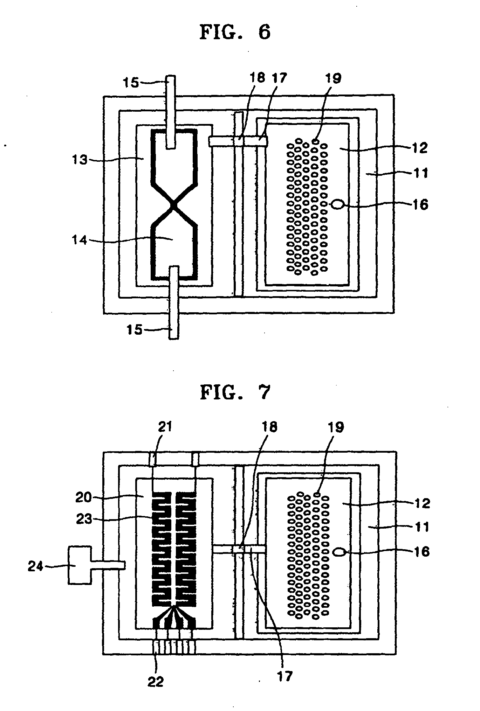

Examples of nanopore system and micro-PCR system are illustrated in FIG. 6 and 7.

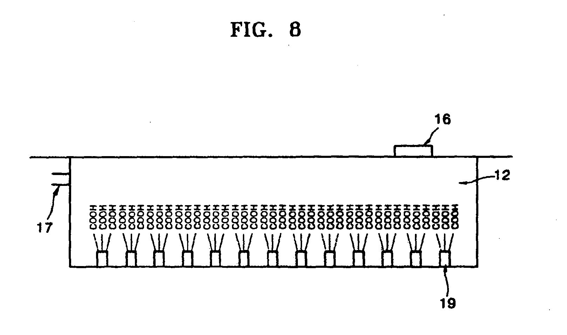

Referring to FIGS. 6 and 7, nanopore system and micro-PCR system include a sample pretreatment chamber 12 (FIG. 6 and 7). The sample pretreatment chamber 12 includes a carboxyl group-coated solid support. An enlarged view of an example of the sample pretreatment chamber 12 is illustrated in FIG. 8. Referring to FIGS. 6 through 8, the sample pretreatment chamber 12 includes a sample inlet 16 and carboxyl group-coated pillars 19. A sample loaded into the sample pretreatment chamber 12 via the sample inlet 16 is subjected to removal of PCR inhibitors, and then transferred to the Nanopore chamber 13 and 14 (FIG. 6) and PCR chamber 20 (FIG. 7) via a channel 17. The channel 17 may include a valve 18 capable of adjusting a sample flux. If the sample pretreatment chamber 12 includes carboxyl group-coated beads, a filter for filtering the beads may be installed in the channel 17. In this case, the beads on which PCR inhibitors are attached may not pass through the filter.

The PCR chamber 20 is not particularly limited provided that can perform common micro-PCR. However, as shown in FIG. 7, the Nanopore chamber 13 and 14 (FIG. 6) and the PCR chamber 20 (FIG. 7) may include negative (upper) and positive (down) electrodes 15 (FIG.6) for DNA translocation and common temperature controlling elements such as heating electrodes 21 and 22 (FIG. 7), a cooler 24 (FIG. 7), and a heating wire 23 (FIG. 7). In this case, since heat treatment is performed for PCR amplification, the PCR duration of an insoluble sample can be reduced.

As described above, according to a micro-PCR system of the present invention, a PCR inhibitor can be rapidly removed in a pretreatment chamber containing carboxyl group-coated solid supports, and a thus-pretreated sample can be directly transferred to a PCR chamber. That is, PCR inhibitor removal and PCR can be performed at the same time in one system. Therefore, the PCR inhibitor removal and PCR performed by a micro-PCR system of the present invention are simple, cost-effective, and time non-consuming, and do not require a separate reagent, unlike a conventional technique.

Hereinafter, the present invention will be described more specifically with reference to the following examples.

### Example 1

### Evaluation of serum IgG reduction after pretreatment with carboxyl group-coated beads

To evaluate a reduction of IgG known as a major PCR inhibitor in serum after pretreatment with carboxyl group-coated beads, the following experiment was performed.

60 µℓ of a human serum sample was loaded in a test tube and IgG concentration was measured using a fluorometer (Spectra MAX GEMINI XS).

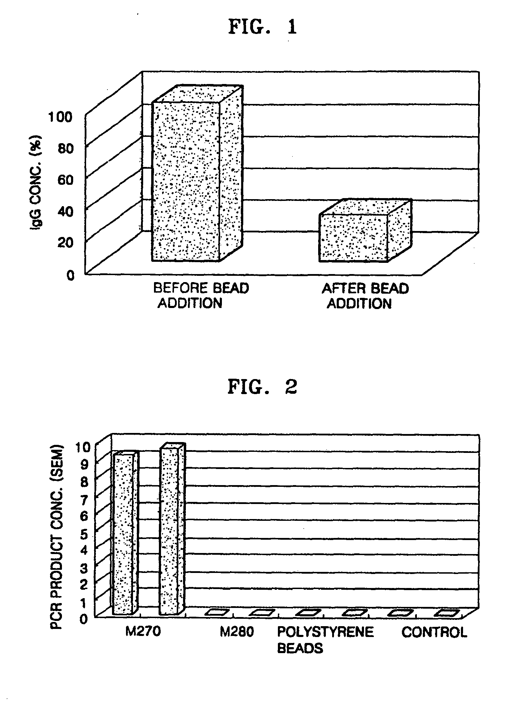

Then, 30 µℓ of a carboxyl group-coated bead solution (Dynabeads M270, bead size: 2.8 micrometers, 4 * 10⁹ beads/ml, Dynal MPC) was added to the serum sample and mildly stirred, and IgG concentration was again measured using a fluorometer (Spectra MAX GEMINI XS). A comparative result for IgG concentrations before and after addition of the M270 beads is illustrated in FIG. 1. Referring to FIG. 1, after addition of the M270 beads, the IgG concentration was reduced by about 71%.

### Example 2

### Evaluation of PCR yield according to the type of beads

After pretreatment with several types of beads, PCR for serum nucleic acids was performed. Targets used in the test were non-infectious, defective rHBV particles (obtained from Yonsei Univ.).

In detail, to three test tubes, there were respectively added three samples obtained by adding 30 *µ*ℓ of each of carboxyl group-coated beads M270, streptavidin-coated beads M280 (Dynal MPC), and uncoated polystyrene beads to a mixture of 60 *µ*ℓ of a human serum sample and 10 *µ*ℓ of the rHBV particles (10⁶ copies/ml). The three test tubes containing the different beads were centrifuged at 12,000 rpm for one minute, supernatants were separated, and then PCR for the supernatants was performed as follows.

The following PCR primers were used:
Primer A (5-AGTGTGGATTCGCACTCCT-3) (SEQ ID NO: 1); and
Primer B (5-GAGTTCTTCTTCTAGGGGACCTG-3) (SEQ ID NO: 2).

PCR was performed using Taq polymerase (Takara, Solgent, Korea) as follows: 50 cycles [(50 cycles for pre-denaturation at 95°C for 1 minute, denaturation at 95°C for 5 seconds, and annealing and extension at 62°C for 15 seconds), extension at 72°C for 15 seconds, and additional extension at 72°C for 1 minute].

Amplified DNAs were analyzed using the DNA 500 assay reagent sets in the Agilent 2100 BioAnalyzer [2100] (Agilent Technologies, Palo Alto, CA).

After PCR was terminated, the concentration of PCR products was measured and the results are shown in FIG. 2. PCR result with no pretreatment with beads was used as control. Referring to FIG. 2, PCR products were observed in the M270 beads-pretreated sample, whereas no PCR products were observed in the other samples. That is, only the M270 beads exhibited PCR inhibitor removal capability.

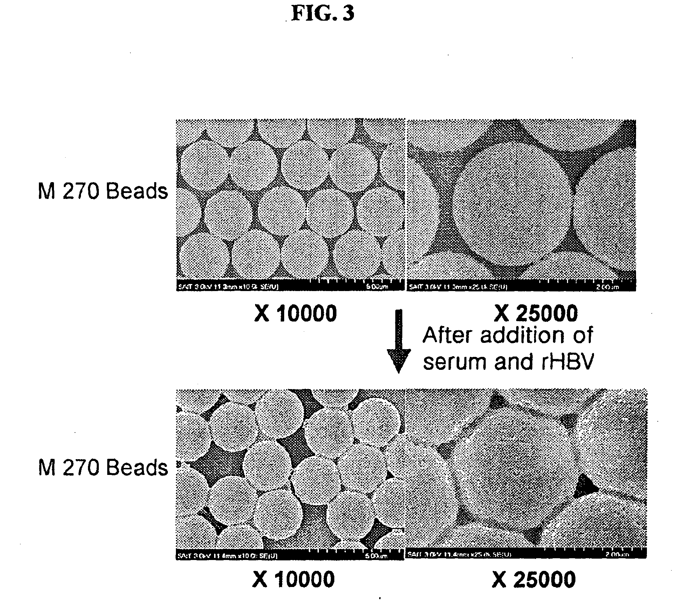

Further, the morphological variation of carboxyl group-coated beads M270 added to an IgG-containing serum sample was observed by a Scanning Electron Microscope (SEM) and the SEM image is shown in FIG. 3. Referring to FIG. 3, adsorption of materials to surfaces of the M270 beads was observed.

### Example 3

To compare purification results by a conventional QIAamp UltraSens Virus kit (Qiagen) and by a carboxyl group-coated bead of the present invention, the following experiment was performed.

60 µℓ of human serum was mixed with 10 µℓ of rHBV particles (10⁶ copies/ml) and then 30 *µ*ℓ of carboxyl group-coated beads M270 (4 * 10⁹ beads/ml, Dynal MPC) were added thereto. The resultant sample mixture was mildly stirred and centrifuged at 12,000 rpm for one minute. A supernatant was separated and PCR for the supernatant was performed in the same manner as in Example 2. The above experiment was repeated three times.

On the other hand, a sample mixture was prepared in the same manner as above in the absence of beads and purified by the QIAamp UltraSens Virus kit (Qiagen) and then PCR was performed in the same manner as in Example 2. The experiment was repeated three times.

The concentration of PCR products for the two experiments was measured by a spectrometer and the results are shown in FIG. 4. As can be seen from FIG. 4, a method of the present invention using a carboxyl group-coated bead is simplified and exhibits a more excellent PCR inhibitor removal effect, relative to a conventional method.

According to a nucleic acid amplification inhibitor removal method and a PCR system of the present invention, nucleic acid amplification inhibitors can be easily removed without additional processes and equipment. The nucleic acid amplification inhibitor removal method and the PCR system can be applied to all kinds of amplification reactions anywhere at any time by those of ordinary skilled in the art. In addition, the nucleic acid amplification inhibitor removal method and the PCR system are more easy and efficient relative to a conventional technique.
## Claims

1. A method of removing a nucleic acid amplification inhibitor from a biological sample, the method comprising contacting the biological sample to a carboxyl group-coated solid support, wherein the nucleic acid amplification inhibitor is adsorbed by the carboxyl group-coated solid support.

2. The method of claim 1, wherein the solid support is in the form of a plate, a bead, or a pillar.

3. The method of claim 1, wherein the solid support is made of glass, silicone, or polymer.

4. The method of claim 3, wherein the polymer is selected from the group consisting of polyethylene, polypropylene, polyacrylate, polyurethane, and polystyrene.

5. The method of claim 1, further comprising filtering the solid support contacted to the biological sample.

6. The method of claim 1, wherein the nucleic acid amplification is PCR.

7. A micro-PCR system comprising:
a sample pre-treatment chamber comprising a carboxyl group-coated solid support;
a PCR chamber; and
a channel connecting the sample pretreatment chamber and the PCR chamber,
wherein a nucleic acid amplification inhibitor binds to the carboxyl group-coated solid support in the sample pre-treatment chamber, and wherein the supernatant from the sample pre-treatment chamber is channeled into the PCR chamber.

8. The micro-PCR system of claim 7, wherein the solid support is in the form of a plate, a bead, or a pillar.

9. The micro-PCR system of claim 7, wherein the solid support is made of glass, silicone, or polymer.

10. The micro-PCR system of claim 9, wherein the polymer is selected from the group consisting of polyethylene, polypropylene, polyacrylate, polyurethane, and polystyrene.

11. The micro-PCR system of claim 7, wherein the channel comprises a valve.

## Patentansprüche

1. Verfahren zum Entfernen eines Nukleinsäureamplifikationsinhibitors aus einer biologischen Probe, wobei das Verfahren das in Kontakt bringen der biologischen Probe mit einem mit einer Carboxylgruppe beschichteten festen Träger umfasst, wobei der Nukleinsäureamplifikationsinhibitor durch den mit einer Carboxylgruppe beschichteten festen Träger adsorbiert wird.

2. Verfahren nach Anspruch 1, wobei der feste Träger in Form einer Platte, einer Perle oder einer Säule vorliegt.

3. Verfahren nach Anspruch 1, wobei der feste Träger aus Glas, Silicon oder Polymer gefertigt ist.

4. Verfahren nach Anspruch 3, wobei das Polymer ausgewählt ist aus der Gruppe bestehend aus Polyethylen, Polypropylen, Polyacrylat, Polyurethan und Polystyrol.

5. Verfahren nach Anspruch 1, außerdem umfassend das Abfiltrieren des mit der biologischen Probe in Kontakt gebrachten festen Trägers.

6. Verfahren nach Anspruch 1, wobei es sich bei der Nukleinsäureamplifikation um eine PCR handelt.

7. Mikro-PCR-System, umfassend:
eine Probenvorbehandlungskammer, die einen mit einer Carboxylgruppe beschichteten festen Träger umfasst;
eine PCR-Kammer; und
einen Kanal, der die Probenvorbehandlungskammer und die PCR-Kammer verbindet,
wobei ein Nukleinsäureamplifikationsinhibitor an den mit einer Carboxylgruppe beschichteten festen Träger in der Probenvorbehandlungskammer bindet und wobei der Überstand von der Probenvorbehandlungskammer in die PCR-Kammer geleitet wird.

8. Mikro-PCR-System nach Anspruch 7, wobei der feste Träger in Form einer Platte, einer Perle oder einer Säule vorliegt.

9. Mikro-PCR-System nach Anspruch 7, wobei der feste Träger aus Glas, Silicon oder Polymer gefertigt ist.

10. Mikro-PCR-System nach Anspruch 9, wobei das Polymer ausgewählt ist aus der Gruppe bestehend aus Polyethylen, Polypropylen, Polyacrylat, Polyurethan und Polystyrol.

11. Mikro-PCR-System nach Anspruch 7, wobei der Kanal ein Ventil umfasst.

## Revendications

1. Procédé d'élimination d'un inhibiteur d'amplification d'acide nucléique d'un échantillon biologique, le procédé comprenant la mise en contact de l'échantillon biologique avec un support solide revêtu d'un groupe carboxyle, dans lequel l'inhibiteur d'amplification d'acide nucléique est adsorbé par le support solide revêtu d'un groupe carboxyle.

2. Procédé selon la revendication 1, dans lequel le support solide est sous la forme d'une plaque, d'une bille ou d'une colonne.

3. Procédé selon la revendication 1, dans lequel le support solide est constitué de verre, de silicone ou de polymère.

4. Procédé selon la revendication 3, dans lequel le polymère est choisi dans le groupe consistant en le polyéthylène, le polypropylène, le polyacrylate, le polyuréthane et le polystyrène.

5. Procédé selon la revendication 1, comprenant en outre la filtration du support solide mis en contact avec l'échantillon biologique.

6. Procédé selon la revendication 1, dans lequel l'amplification d'acide nucléique est une PCR.

7. Système micro-PCR comprenant :
une chambre de prétraitement d'échantillon comprenant un support solide revêtu d'un groupe carboxyle ;
une chambre PCR ; et
un canal reliant la chambre de prétraitement d'échantillon et la chambre PCR,
dans lequel un inhibiteur d'amplification d'acide nucléique se lie au support solide revêtu d'un groupe carboxyle dans la chambre de prétraitement d'échantillon et dans lequel le surnageant de la chambre de prétraitement d'échantillon est acheminé par canal dans la chambre PCR.

8. Système micro-PCR selon la revendication 7, dans lequel le support solide est sous la forme d'une plaque, d'une bille ou d'une colonne.

9. Système micro-PCR selon la revendication 7, dans lequel le support solide est constitué de verre, de silicone ou de polymère.

10. Système micro-PCR selon la revendication 9, dans lequel le polymère est choisi dans le groupe consistant en le polyéthylène, le polypropylène, le polyacrylate, le polyuréthane et le polystyrène.

11. Système micro-PCR selon la revendication 7, dans lequel le canal comprend une soupape.


<110> Samsung Electronics Co., Ltd
<120> Method of removing inhibitors of amplification of nucleic acid from sample
<130> pn059300
<160> 2
<170> KopatentIn 1.71
<210> 1
   <211> 19
   <212> DNA
   <213> Artificial Sequence
<220>
   <223> primer
<400> 1
   agtgtggatt cgcactcct 19
<210> 2
   <211> 23
   <212> DNA
   <213> Artificial Sequence
<220>
   <223> primer
<400> 2
   gagttcttct tctaggggac ctg 23